# EUROPEAN PATENT APPLICATION

(11) **EP 2 042 190 A1**
(43) Date of publication of application: **01.04.2009**
(21) Application number: 07743822.4
(22) Date of filing: 21.05.2007
(51) Int. Cl.: A61K 38/43, A61K 35/12, A61K 35/30, A61P 9/00, A61P 25/00

(54) **AMELIORATING AGENT FOR BRAIN DAMAGE**

(30) Priority: 19.05.2006 JP 2006139843
(71) Applicant: Aichi Prefecture, Aichi 460-8501 (JP); SEIKAGAKU CORPORATION, Chiyoda-ku, Tokyo 100-0005 (JP)
(72) Inventor: OOHIRA, Atsuhiko, Aichi-gun Aichi 480-1195 (JP); SATO, Yoshiaki, 40530 Göteborg (SE); NAKANISHI, Keiko, Kasugai-shi Aichi 480-0392 (JP); MAEDA, Hiroshi, Higashiyamato-shi Tokyo 207-0021 (JP)
(74) Representative: Elsy, David
(86) International application number: PCT/JP2007/060388
(87) International publication number: WO 2007/136042

(57) **Abstract**

The present invention provides a pharmaceutical composition for ameliorating brain damage induced by oxygen deficiency, comprising a chondroitin sulfate-degrading enzyme and at least one of a neural stem cell(s) and a neural progenitor cell(s), as active ingredients.

## Description

### Field of the Invention

The present invention relates to an agent or the like for ameliorating and suppressing brain damage induced by oxygen deficiency.

### Background Art

Ischemic brain disease such as thrombotic cerebral infarction or embolic cerebral infarction, which is cerebral stroke known as one of three major causes of death in Japan, has become a problem from the viewpoint of lifestyle habits of modern people who eat too much food and exercise little, and many studies have been made for treating the diseases. Meanwhile, in spite of dramatic improvement of survival rate and prognosis of premature infants and diseased newborn infants by progress in medical technology, a high proportion of newborn infants still suffer from hypoxic-ischemic encephalopathy (HIE) which is one of ischemic brain diseases, and the morbidity rate is 2 to 4 per 1,000 newborn infants. HIE is a main cause of cerebral palsy, but at present, an effective medical therapy is only brain hypothermia treatment. Moreover, such diseases including cerebral palsy due to perinatal brain damage are increasing as the number of low-birth-weight babies increases in recent years, which is a problem in the field of neonatal care. Therefore, development of a method of suppressing such brain damage due to oxygen deficiency has been desired.

On the other hand, in recent years, attention has been focused on regenerative medicine using stem cells as a novel therapy based on the viewpoint of "compensation of lost cells by regeneration", and extensive studies have been made on many diseases and organ damages, and clinical applications thereof have been started.
Studies on regenerative medicine in the nervous system have revealed that transplantation of neural stem/progenitor cells to a damage area in the spinal cord of an adult rat or monkey is effective for repairing the damage area in the spinal cord and that administration of chondroitinase ABC in combination with transplantation of neural stem/progenitor cells to a damage area in the spinal cord of an adult rat caused by a bruise is more effective for regeneration of the damage area in the spinal cord (Non-Patent Document 1). However, those effects are for the spinal cord, and effects on the brain have not been reported. In addition, there is no report on application and effects in the brain of a newborn animal, whose brain is in the course of development to an adult brain and is light compared with the brain of an adult and whose neural circuit is underdeveloped. Moreover, there has been reported that use of a nerve graft and a chondroitin sulfate proteoglycan-degrading enzyme is useful for promoting axon elongation in the Schwann cell basal lamina and for repairing a cut nerve (Patent Document 1). However, this report has been made based on the results of an experiment on treatment of nerve rupture that requires surgical treatments in the peripheral nerve using a graft previously treated with chondroitin sulfate proteoglycan, and there is no report on application to a damage induced by oxygen deficiency in the central nerve.

Patent Document 1: JP 2005-500375 A
Non-Patent Document 1: European Journal of Neuroscience, Federation of European Neuroscience Societies, 2005, vol. 22, p 3036-3046

### Disclosure of the Invention

An object of the present invention is to provide a tool for effectively ameliorating brain damage induced by insufficient supply of oxygen to brain cells.

The inventors of the present invention have made extensive studies on a treatment for hypoxic and ischemic brain damage from the viewpoint of regenerative medicine. As a result, the inventors have found that the brain damage can be ameliorated by using both a chondroitin sulfate-degrading enzyme and a neural stem/progenitor cell(s) in the brain damage induced by oxygen deficiency (hypoxic condition), thus completed the present invention.

That is, the present invention provides the followings.
(1) A medicament for ameliorating brain damage induced by oxygen deficiency, which comprises a chondroitin sulfate-degrading enzyme and at least one of a neural stem cell(s) and a neural progenitor cell(s), as active ingredients.
(2) The medicament according to (1), wherein the brain damage induced by oxygen deficiency is induced by hypoxia-ischemia.
(3) The medicament according to (1) or (2), wherein the chondroitin sulfate-degrading enzyme is one or a plurality of enzymes selected from the group consisting of chondroitinase ABC, chondroitinase AC, and hyaluronidase.
(4) The medicament according to (1) or (2), wherein the chondroitin sulfate-degrading enzyme degrades chondroitin sulfate composed of a constituent disaccharide unit of chondroitin sulfate in which a hydroxyl group at 4-position of N-acetylgalactosamine is sulfated.
(5) The medicament according to (1) or (2), wherein the chondroitin sulfate-degrading enzyme is chondroitinase ABC or chondroitinase AC enzymes.
(6) The medicament according to (1) or (2), wherein the chondroitin sulfate-degrading enzyme is chondroitinase ABC.
(7) The medicament according to any one of (1) to (6), wherein the neural stem cell(s) and neural progenitor cell(s) are derived from a brain.
(8) The medicament according to any one of (1) to (6), wherein the neural stem cell(s) is a cultured neural stem cell(s) and neural progenitor cell(s) is a cultured neural progenitor cell(s).
(9) The medicament according to any one of (1) to (8), wherein the medicament is applied to a brain in the course of development to an adult brain.
(10) The medicament according to any one of (1) to (8), wherein the medicament is applied to a brain of a neonate or a newborn.
(11) The medicament according to any one of (1) to (10), which is an agent for ameliorating neonatal hypoxic-ischemic encephalopathy.
(12) The medicament according to any one of (1) to (10), which is an agent for reducing a cerebral infarction area.
(13) The medicament according to any one of (1) to (10), which is an agent for protecting a brain tissue.
(14) An auxiliary agent for ameliorating brain damage induced by oxygen deficiency, which comprises a chondroitin sulfate-degrading enzyme as an active ingredient.
(15) Use of a chondroitin sulfate-degrading enzyme and at least one of a neural stem cell(s) and a neural progenitor cell(s) in manufacturing the medicament according to any one of (1) to (13).
(16) Use of a chondroitin sulfate-degrading enzyme in manufacturing the auxiliary agent for ameliorating brain damage according to (14).
(17) A kit for ameliorating brain damage induced by oxygen deficiency, which comprises a chondroitin sulfate-degrading enzyme and at least one of a neural stem cell(s) and a neural progenitor cell(s).
(18) A method of treating brain damage induced by oxygen deficiency, which comprises applying a chondroitin sulfate-degrading enzyme and at least one of a neural stem cell(s) and a neural progenitor cell(s) to a brain damage area.

### Brief Description of the Drawings

Fig. 1 is a drawing (photograph) showing states of brain damage induced by oxygen deficiency in 7-day-old SD rats. The abbreviation "CX" means a cerebral cortex. The abbreviation "CA2" means a hippocampal CA2 region. The abbreviation "CA3" means a hippocampal CA3 region. The abbreviation "Den" means a dentate hilus. The abbreviation "TH" means a thalamus. The abbreviation "ST" means a striatum.
Fig. 2 is a graph showing effects (brain weight ratios) provided by use of both neural stem cells and C-ABC.
Fig. 3 is a graph showing effects (non-infarction area ratio) provided by use of both neural stem cells and C-ABC.

### Description of the Preferred Embodiments

Hereinafter, the present invention is described in detail by way of embodiments of the invention.
The term "neural stem/progenitor cell(s)" as used herein includes a neural stem cell(s) alone, a neural progenitor cell(s) alone, and "a mixture of neural stem cell(s) and neural progenitor cell(s)", and the term "neural progenitor cell(s)" used herein means a precursor cell(s) of a neuron.

The chondroitin sulfate-degrading enzyme of the present invention is used together with a neural stem/progenitor cell(s) to be hereinafter described to effectively ameliorate or suppress brain damage induced by oxygen deficiency.
As described above, the chondroitin sulfate-degrading enzyme to be used in the present invention is not particularly limited as long as the enzyme is used for brain damage induced by oxygen deficiency (hypoxic condition) and can be used together with the neural stem/progenitor cell(s) at the time of application.

Various chondroitin sulfate-degrading enzymes are known, and specific examples of the enzymes include enzymes that degrade chondroitin sulfate by an elimination reaction and enzymes that hydrolyze chondroitin sulfate. The former is classified as a lyase, and examples of thereof include chondroitinase ABC, chondroitinase AC enzymes (chondroitinase ACI, chondroitinase ACII, and chondroitinase ACIII), chondroitinase C, and chondroitin sulfate ABC exolyase. Examples of the latter include hyaluronidase and a human-derived chondroitin sulfate-degrading enzyme described in JP 3759774 B, and examples of the hyaluronidase include hyaluronidase (HYAL4) described in Trends in Glycoscience and glycotechnology 89 (2004) pp171-185. All of the enzymes can be used in the present invention, and the enzymes may be used alone or two or more of them in combination may be used.

Of those, the chondroitin sulfate-degrading enzyme to be used in the present invention is preferably chondroitinase ABC, chondroitinase AC enzymes, hyaluronidase, or the human-derived chondroitin sulfate-degrading enzyme described in JP 3759774 B, more preferably a lyase, and most preferably chondroitinase ABC or chondroitinase ACII. In a brain, there is a large amount of chondroitin sulfate (chondroitin sulfate A) composed of a constituent disaccharide unit of chondroitin sulfate including D-glucuronic acid and N-acetylgalactosamine where a hydroxyl group at 4-position of N-acetylgalactosamine is sulfated, and in the present invention, an enzyme having the activity to degrade such chondroitin sulfate is preferably selected.

The human-derived chondroitin sulfate-degrading enzyme described in JP 3759774 B specifically means an enzyme having the following physical and chemical properties (1) to (5).
(1) Reaction: the enzyme hydrolyzes an N-acetylhexosaminide bond of glycosaminoglycan.
(2) Substrate specificity: the enzyme reacts with chondroitin sulfate and does not react with hyaluronic acid, keratan sulfate, heparan sulfate, and heparin at pH 5. The enzyme reacts with chondroitin sulfate and hyaluronic acid at pH 3.5.
(3) Optimum reaction pH: about pH 5 (substrate: chondroitin sulfate, buffer: 50 mM citric acid-Na₂HPO₄ buffer containing 0.15 M NaCl, temperature: 37°C)
(4) Isoelectric point: about pH 5
(5) Molecular weight: about 36 kDa in sodium dodecyl sulfate-polyacrylamide gel electrophoresis on a polyacrylamide gel obtained by copolymerizing chondroitin sulfate chains each having an allyl group on its end.

The origin of the chondroitin sulfate-degrading enzyme to be used in the present invention is not particularly limited as long as the enzyme is the above-mentioned chondroitin sulfate-degrading enzyme. Examples of the origin of the enzyme include human, animals, and bacteria. The enzyme further includes not only naturally-derived enzymes but also genetically-engineered recombinant enzymes and the like. Any of those enzymes can be used in the present invention.

Of those, an enzyme that is preferably used in the present invention includes, for example, chondroitinase ABC derived from *Proteus vulgaris,* chondroitinase ACI derived from *Flavobacterium heparinum*, chondroitinase ACII derived from *Arthrobacter aurescens*, chondroitinase ACII derived from *Flavobacterium* sp. Hp 102 strain, chondroitinase ACIII derived from *Flavobacterium* sp. Hp 102 strain, chondroitinase C derived from *Flavobacterium* sp. Hp 102 strain, and chondroitin sulfate ABC exolyase described in J. Biol. Chem., 272, pp. 9123-9130 (1997). Of those, chondroitinase ABC derived from *Proteus vulgaris* and chondroitinase ACII derived from *Arthrobacter aurescens* are preferably used.

The term "derived from" used herein for a chondroitinase (chondroitin sulfate-degrading enzyme) means that the enzyme originates from a living being that originally has a gene encoding the chondroitinase. Therefore, for example, chondroitinase ABC derived from *Proteus vulgaris* includes not only an enzyme produced by *Proteus vulgaris* itself but also an enzyme produced in other cells by a genetic engineering technique based on a gene encoding chondroitinase ABC obtained from *Proteus vulgaris.* If necessary, the enzyme also includes an enzyme having a mutation introduced by a genetic engineering process. Examples thereof include Chondroitinase ABCI Mutants described in WO 2007/038548, mutants described in WO 2004/110360, and fusion proteins described in WO 2004/110359.

The neural stem/progenitor cell of the present invention is used together with the above-mentioned chondroitin sulfate-degrading enzyme to ameliorate or suppress brain damage induced by oxygen deficiency. The neural stem/progenitor cell to be used in the present invention is not particularly limited as long as the cell is used for brain damage induced by oxygen deficiency (hypoxic condition) as described above and used together with the above-mentioned chondroitin sulfate-degrading enzyme at the time of application and has self-proliferation ability and/or differentiation ability. Such neural stem/progenitor cell includes neural stem/progenitor cells that are generally used in the field of regenerative medicine.
In general, the "neural stem cell" is defined as a cell having self-proliferation ability and having ability to differentiate into cells that constitute the central nerve system such as neurons, astrocytes, and oligodendrocytes, while the "neural progenitor cell" is defined as an undifferentiated cell obtained by division of a neural stem cell but is considered by a person skilled in the art to have ability to differentiate after cell division about once or twice. Note that differentiation proceeds in the following order: a neural stem cell → a neural progenitor cell → a (mature) neuron.

The kinds of the neural stem/progenitor cell to be used in the present invention can be appropriately determined by a person skilled in the art depending on kinds of an individual where the neural stem/progenitor cell is transplanted, degree of development of brain, age, damage area, degree of the damage of the individual and etc. For example, the neural stem cell to be used may be one derived from bone marrow, peripheral blood, brain, spinal cord, umbilical cord blood, dental pulp, blood vessel, or the like. Of those, the neural stem cell is preferably one derived from a nervous tissue such as spinal cord, brain, or the like, particularly preferably one derived from brain. For example, in the case of transplantation in a newborn mammal, a neural stem cell derived from the fetal brain primordium is particularly preferably used.
The term "mammal" used herein includes both a human and mammal other than a human. In addition, the term "newborn" or "neonate" used herein means an individual immediately after birth. For example, in the case of a mouse or rat, the term means an individual about seven to ten days from birth, while in the case of a human, the term means an individual less than one month from birth.

The neural stem/progenitor cell to be used in the present invention may be cultured in advance. The culture medium, culture conditions, and the like are not particularly limited as long as the neural stem/progenitor cell can be obtained in an amount and quality sufficient for transplantation in consideration of kinds or origin of the neural stem/progenitor cell, damage area where the cell is transplanted, and the like, and can be determined by a person skilled in the art. For example, a medium and conditions for proliferation of a neural stem/progenitor cell that is generally used in the field of regenerative medicine may be employed, but in the present invention, culture is preferably carried out under conditions where cultured cells does not differenciate. For example, the medium may be a basal medium containing some essential components (inorganic salts, carbohydrates, hormones, essential amino acids, vitamins, etc.) necessary for maintenance and proliferation of cells, and examples of the medium include Dulbecco's Modified Eagle's Medium (DMEM), F-12 medium, and Neurobasal medium. In addition, a medium further supplemented with a growth factor is preferable, and examples of the growth factor include a basic fibroblast growth factor (bFGF), an epidermal growth factor (EGF), and a leukocyte migration inhibitory factor (LIF). Moreover, if necessary, the composition of the medium can be appropriately selected by a person skilled in the art: such as adding a medium additive B27 or N2 (manufactured by Gibco) to promote proliferation. Examples of the culture conditions include culture at 37°C for 10 to 20 days, but the conditions are preferably determined by a person skilled in the art while confirming culture states and the like.

In the present invention, the above-mentioned chondroitin sulfate-degrading enzyme and neural stem/progenitor cells are applied in combination to the damage area in order to ameliorate brain damage induced by oxygen deficiency (hypoxic condition) and to suppress progress of the brain damage. The method of "applying" is not particularly limited as long as the method enables coexistence of the chondroitin sulfate-degrading enzyme and neural stem/progenitor cell(s) in the damage area.
For the neural stem/progenitor cell, for example, a method of transplanting a neural stem/progenitor cell(s) to a desired area that is generally used in the field of regenerative medicine may be employed. More specifically, there can be exemplified, for example, a method of transplanting a neural stem/progenitor cell(s) to an area of interest by: suspending neural stem/progenitor cells in phosphate buffered saline; and adding/injecting the resultant cell suspension to the area.
Meanwhile, for the chondroitin sulfate-degrading enzyme, for example, a method of administering a solution of a chondroitin sulfate-degrading enzyme by injection may be exemplified.

In the present invention, the phrases "using a chondroitin sulfate-degrading enzyme together with a neural stem/progenitor cell(s)" and "using a neural stem/progenitor cell(s) together with a chondroitin sulfate-degrading enzyme" mean "simultaneous use" within a scope generally understood by a person skilled in the art and are not intended to include a case where one treatment is performed for a damage area and then stopped, and the other treatment is performed after a lapse of a certain time. That is, a series of treatments for a damage area may be performed continuously, and the phrases include, for example, a case where a chondroitin sulfate-degrading enzyme is administered after transplantation of neural stem/progenitor cells, a case where neural stem/progenitor cells are transplanted after administration of a chondroitin sulfate-degrading enzyme, and a case where both are transplanted (administered) simultaneously. According to the application (administration) method, the present invention can provide a method of treating brain damage induced by oxygen deficiency. The term "treatment" used herein means therapy and amelioration of brain damage induced by oxygen deficiency, suppression of progress of brain damage and the like.

In the present invention, a brain damage induced by oxygen deficiency (hypoxic condition) is not particularly limited as long as the brain damage is caused by a reduction in the oxygen amount in the brain or the oxygen supply amount to cerebral tissues. Examples of the brain damage include thrombus in the brain blood vessel, embolus in the brain blood vessel, intracerebral hemorrhage caused by rupture of the brain blood vessel, compression of the brain blood vessel, poor blood circulation caused by abnormality in a circulatory system, obstruction in a respiratory system, and low-oxygen and ischemic conditions due to asphyxia, intracerebral hemorrhage, or the like during obstetric delivery or at birth. Examples of the brain damage further include a brain damage caused by lowered oxygen supply due to external environment factors such as fire and drowning in water. Of those, brain damage caused by hypoxia-ischemia is particularly preferable.

As described above, when the chondroitin sulfate-degrading enzyme and neural stem/progenitor cell of the present invention are used together, brain damage induced by oxygen deficiency (hypoxic condition) is effectively ameliorated and suppressed. Therefore, the present invention can be applied for protection of brain and various diseases due to oxygen deficiency (hypoxic condition). Examples of the diseases include transient ischemic attack syndrome (TIA syndrome), ischemic cerebrovascular disease, neonatal hypoxic-ischemic encephalopathy, thrombosis due to atherosclerosis, atherothrombotic lesion in the internal carotid artery (including branches), cerebral infarction, cerebral embolism, intracerebral hemorrhage, subarachnoid hemorrhage, hypertensive encephalopathy, moyamoya disease, cerebral vein/cerebral venous sinus obstruction, systemic hypotension, anoxic-ischemic encephalopathy, traumatic cerebrovascular obstruction, cerebral concussion, cerebral contusion, epidural hematoma, subdural hematoma, cerebrovascular diseases (such as intracerebral hemorrhage, cerebral infarction, subarachnoid hemorrhage, and hypertensive encephalopathy), encephalitis, and brain tumor.

The present invention is particularly suitable for damage in the brain of a newborn or neonate, which is in the course of development to an adult and has neurons with different differentiation degrees and insufficiently functioned neural circuits, and the present invention is very useful for brain disorders and the like induced by asphyxia or brain ischemia during obstetric delivery or at birth and perinatal intracerebral hemorrhage. For example, the present invention is particularly suitable as an agent for ameliorating neonatal hypoxic-ischemic encephalopathy.
As described in Examples below, the present invention provides effects such as suppression of enlargement of a cerebral infarction area induced by oxygen deficiency (hypoxic condition), reduction or amelioration of the cerebral infarction area, and regeneration or amelioration of a cerebral nerve tissue by an increase in the brain weight, and the present invention suggests probability of recovery of brain functions.

The usage and dosage in the case where the present invention is used for therapy of a disease may be appropriately determined by a person skilled in the art in consideration of symptom of an individual to be applied (such as a patient suffering from any of the above-mentioned diseases), state of brain damage, age, body weight, etc. As the usage, a method of applying a chondroitin sulfate-degrading enzyme to a damage area together with a neural stem/progenitor cell(s) in accordance with the above-mentioned method of transplanting the neural stem/progenitor cells and method of administering the chondroitin sulfate-degrading enzyme is included. Note that the dosage varies depending on the age and body weight of an individual of interest as noted in the theory of medicines, but examples of the dosage of chondroitinase ABC include 0.0001 unit to 500 units.
Note that "1 unit" of the above-mentioned enzymatic activity is the amount of an enzyme that produces 1 µ mol of an unsaturated disaccharide per minute from chondroitin sulfate C derived from shark cartilage under the condition of 37°C and pH 8.0.

The present invention also includes a pharmaceutical composition or kit to be used for ameliorating brain damage induced by oxygen deficiency (hypoxic condition), which comprises the chondroitin sulfate-degrading enzyme of the present invention and the neural stem/progenitor cell of the present invention as components. The present invention may provide, for example, an agent for ameliorating neonatal hypoxic-ischemic encephalopathy, an agent for reducing a cerebral infarction area, and an agent for protecting a brain tissue, all of which comprises the chondroitin sulfate-degrading enzyme of the present invention and the neural stem/progenitor cell of the present invention as components.
In addition, the present invention may provide an agent for ameliorating neonatal hypoxic-ischemic encephalopathy, which comprises a chondroitin sulfate-degrading enzyme and at least one of a neural stem cell and a neural progenitor cell as active ingredients, and may further provide an agent for reducing a cerebral infarction area and an agent for protecting a brain tissue, all of which comprises a chondroitin sulfate-degrading enzyme and at least one of a neural stem cell and a progenitor cell as active ingredients. As described above, for example, the chondroitin sulfate-degrading enzyme and neural stem/progenitor cell in those agents may be independently produced and packed as long as the method enables coexistence of the chondroitin sulfate-degrading enzyme and neural stem/progenitor cell(s) in the damage area, and those agents may be distributed independently like the above-mentioned agent comprising a chondroitin sulfate-degrading enzyme as an active ingredient or the above-mentioned agent comprising a neural stem/progenitor cell as an active ingredient. That is, the present invention may provide an auxiliary agent for ameliorating brain damage induced by oxygen deficiency, which comprises a chondroitin sulfate-degrading enzyme as an active ingredient.

### Examples

Hereinafter, the present invention will be described in detail by referring to Examples. However, the Examples are not intended to limit the technical scope of the present invention.

### <Referential Example 1>

### <Culture of neural stem cell derived from fetal rat cerebrum>

14-day-old fetal rats obtained from a pregnant rat (EGFP transgenic rat, manufactured by Japan SLC, Inc.) transformed by introduction of green fluorescent protein (GFP) gene were used. GFP-labeled neural stem cells were obtained from brain primordial of each fetal rat of the transformant. The resultant labeled neural stem cells were cultured by the neurosphere method, being a method of selectively culturing neural stem cells. Specifically, a single cell suspension prepared by dissociating the labeled neural stem cells in phosphate buffered saline (PBS) was cultured in DMEM/F-12 (1:1) medium at the temperature of 37°C in the presence of growth factors, that is, fibroblast growth factor-2 (FGF-2) and epidermal growth factor (EGF). 6 days after the beginning of culture, neurospheres formed from proliferated labeled neural stem cells were physically dissociated in PBS, to thereby.prepare a single cell suspension again.

### <Referential Example 2>

### <Preparation of neonatal hypoxic-ischemic encephalopathy (HIE) model>

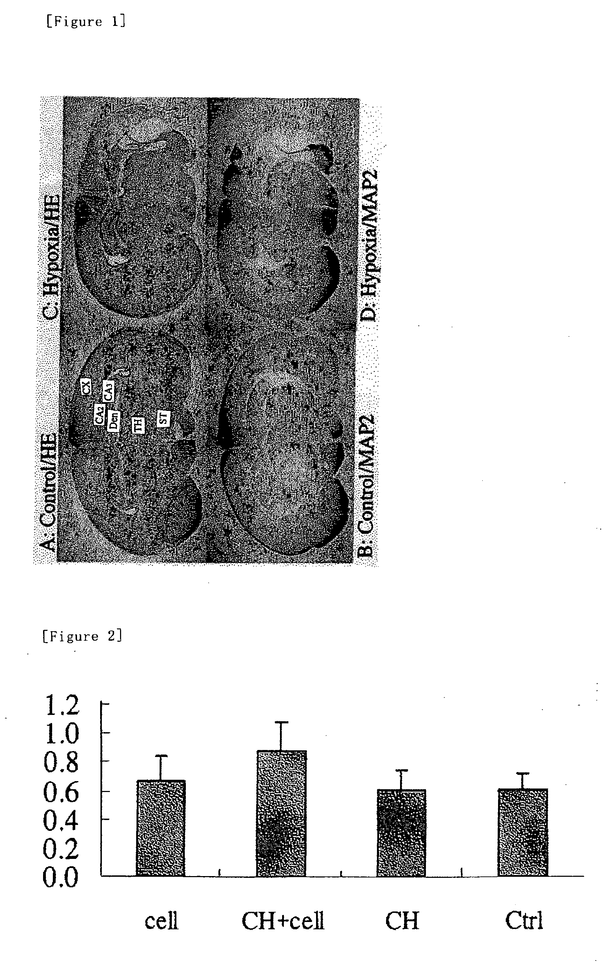

In accordance with the method of Rice et al. (Rice JE 3rd, Vannucci RC, Brierley JB. The influence of immaturity on hypoxic-ischemic brain damage in the rat. Ann Neurol, 9:131-141. 1981.), rats to be used as models of neonatal hypoxic-ischemic encephalopathy (HIE) were prepared from 7-day-old SD rats. Specifically, a rat in which the right common carotid artery was doubly ligated and cut was left in a hypoxic condition of 8% oxygen/92% nitrogen for 2 hours to prepare a rat with HIE only in the right brain.
The resultant rat was sacrificed, and a section was prepared with a portion at a distance of 5 mm from the anterior end of the brain and subjected to hematoxylin-eosin staining (HE staining) and MAP2 staining. As a control, a 7-day-old SD rat that did not undergo the hypoxic condition was also subjected to the staining in the same way as above, and the brain shapes and infarction areas were observed (Fig. 1). The results of hematoxylin-eosin staining and MAP2 staining for the rat with HIE in the right brain are shown in Fig. 1C and Fig. 1D, respectively, while the results of hematoxylin-eosin staining and MAP2 staining for the control rat are shown in Fig. 1A and Fig. 1B, respectively.
As is obvious from Fig. 1, infarctions occurred in wide areas only in the right brain.

### <Example 1>

Rats with HIE only in the right brains, prepared in accordance with Referential Example 2, were divided into three groups, and only neural stem cells, both neural stem cells and chondroitinase ABC, and only chondroitinase ABC were separately administered to the respective groups according to the following procedures. Note that chondroitinase ABC (C-ABC) used is a product manufactured by Seikagaku Corporation, and the numbers of cells administered and the amounts of the enzyme administered were adjusted to be the same for the three groups.
24 hours after preparation of rats with HIE only in the right brain, 5 µl of a solution of the labeled neural stem cells prepared in accordance with Referential Example 1 (5 × 10⁷ cells/mL) was injected in the ventricle of the right cerebrum of the rat with a Hamilton 27-gauge needle using a head clamping device for a newborn rat and a tool for intracerebral injection at the following coordinates: 2 mm anterior to bregma; 2.5 mm right lateral from the bregma; 4 mm deep from the dura mater, to thereby transplant the labeled neural stem cells.
In the same way as above, a solution obtained by dissolving chondroitinase ABC in a BSA-PBS solution, which was obtained by dissolving bovine serum albumin (BSA) at the concentration of 0.1% in phosphate buffered saline (PBS), was prepared so that a chondroitinase ABC concentration was 10 U/mL. 2.5 µl of the solution (10 U/mL) thus obtained and 2.5 µl of a labeled neural stem cell suspension (1 × 10⁸ cells/mL) were mixed and administered.
Moreover, in the same way as above, 5 µl of 5 U/mL chondroitinase ABC was administered.
Rats obtained by administering PBS to the rats with HIE only in the right brains according to Referential Example 2 were defined as controls.

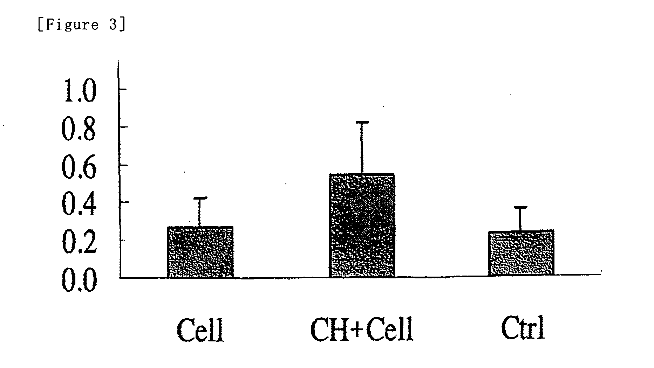

7 days after transplantation of the labeled neural stem cells and/or administration of C-ABC, the rats were sacrificed, and the right and left brains were removed. The effects were evaluated using two indices: the weight ratio of the brains and the area ratio of the non-infarction (undamaged) areas (Table 1). Fig. 2 is a graph showing the weight ratios, and Fig. 3 is a graph showing the area ratios. In Fig. 2, the abbreviates "Cell", "CH + Cell", "CH", and "Ctrl" mean the results of administration of only neural stem cells, administration of both neural stem cells and C-ABC, administration of only C-ABC, and control, respectively, and the abbreviates "Cell", "CH + Cell", and "Ctrl" in Fig.3 have the same meanings as above.
The weight ratios were calculated as follows: the weights of the cerebral cortices of the right and left brains removed were measured. In order to correct individual variabilities of the test rats, a value calculated by dividing the weight of the right cerebral cortex with the weight of the left cerebral cortex in a normal state (weight ratio: wet weight) was used for evaluation (weight ratio = weight of right cerebral cortex/weight of left cerebral cortex).
On the other hand, the area ratios of the non-infarction (undamaged) areas were calculated as follows: the right and left cerebrum removed were fixed with paraformaldehyde for optical microscope, and coronal sections (a frontal section at the brain center part) were prepared with a portion at a distance of 5 mm from the edge of each cerebrum. The sections were subjected to Nissl staining with a fluorescent Nissl staining reagent. After staining, image processing was performed using optical microscope based on Scion image (manufactured by Scion Corporation) to calculate stained areas (non-infarction areas) in the right and left cerebral hemispheres in a quantitative manner, and a value calculated by dividing the stained area in the right cerebral hemisphere with the stained area in the left cerebral hemisphere was used for evaluation (non-infarction area ratio = stained area in right cerebral hemisphere/stained area in left cerebral hemisphere).

**[Table 1]**

| | Neural stem cell | Neural stem cell + chondroitinase ABC | Chondroitinase ABC | Control |
|---|---|---|---|---|
| Weight ratio | 0.67 ± 0.17 | 0.88 ± 0.19 | 0.61 ± 0.14 | 0.61 ± 0.10 |
| Area ratio | 0.27 ± 0.18 | 0.55 ± 0.27 | Not measured | 0.23 ± 0.14 |

In comparison of the weight ratios, the weight of the cerebral hemisphere on the infarction side of the control was found to be only about 60% with respect to the weight of the cerebral hemisphere on the normal side because of degeneration and deficiency of nerve tissues, while in the case of using both neural stem cells and C-ABC, the weight of the cerebral hemisphere on the infarction side was found to be about 90% with respect to the weight of the cerebral hemisphere on the normal side. On the other hand, in the case of transplantation of only neural stem cells and in the case of administration of only C-ABC, the weight ratios were found to be almost the same as the weight ratio of the control. The results suggest that degeneration and deficiency of nerve tissues can be suppressed by co-administering neural stem/progenitor cells and C-ABC to the fetal rat brain subjected to a hypoxic-ischemic treatment.
In addition, according to the results of Nissl staining, which is used for staining non-degenerated regions (normal states) in brain cells, it was found that, in the cases of the control and administration of only neural stem/progenitor cells, the normal regions (non-infarction areas) were about 20 to 30%, while in the case of administration of both neural stem/progenitor cells and C-ABC, the normal region was 50% or more, which revealed that formation of infarct area was effectively suppressed.

The results suggest that use of both transplantation of neural stem cells and administration of chondroitinase ABC is very useful for effectively ameliorating a damage induced by oxygen deficiency (hypoxic condition) in the brain of, particularly, a newborn or neonate that is in the course of development (in the process of growing) to an adult.

### <Example 2>

The inventors of the present invention further made an experiment on chondroitinase ACII derived from *Arthrobacter aurescens*, which is one of chondroitin sulfate-degrading enzymes, instead of chondroitinase ABC.
Rats with HIE only in the right brains prepared in accordance with Referential Example 2 were divided into two groups (4 rats for each group) and subjected to treatment with only neural stem cells and treatment with both neural stem cells and chondroitinase ACII according to the same procedures (including the doses of the cells and enzyme) as described in Example 1 above. Note that chondroitinase ACII used is a product manufactured by Seikagaku Corporation, and the numbers of the cells administered and the dose of the enzyme administered were adjusted to be the same for both the groups.
7 days after transplantation of the labeled neural stem cells and/or administration of chondroitinase ACII, the rats were sacrificed, and the right and left brains were removed. The effects were evaluated using the weight ratio of the brains as an index in the same way as in Example 1 (Table 2).

**[Table 2]**

| | Neural stem cell | Neural stem cell + chondroitinase ACII |
|---|---|---|
| Weight ratio | 0.43 ± 0.08 | 0.50 ± 0.06 |

The results reveal that the treatment with both neural stem cells and chondroitinase ACII is more effective for reducing the cerebral infarction areas compared with the treatment with only neural stem cells.
Since chondroitinase ABC and chondroitinase ACII are chondroitin sulfate-degrading enzymes, the results of the present Examples suggest that the treatment with both neural stem cells and the chondroitin sulfate-degrading enzyme is more effective for ameliorating brain damage induced by oxygen deficiency compared with the treatment with only neural stem cells.

### Industrial Applicability

According to the present invention, brain damage induced by oxygen deficiency (hypoxic condition) or ischemia can be effectively ameliorated and worsening of the damage can be suppressed by transplantation of the neural/progenitor cell(s) simultaneously with administration of a chondroitin sulfate-degrading enzyme. In addition, the present invention is useful for such damage in the brain in the course of development to an adult and is very useful for, for example, brain disorders of a newborn (neonate) induced by asphyxia or brain ischemia during obstetric delivery or at birth and perinatal intracerebral hemorrhage.

## Claims

1. A medicament for ameliorating brain damage induced by oxygen deficiency, which comprises a chondroitin sulfate-degrading enzyme and at least one of a neural stem cell(s) and a neural progenitor cell(s), as active ingredients.

2. The medicament according to Claim 1, wherein the brain damage induced by oxygen deficiency is induced by hypoxia-ischemia.

3. The medicament according to Claim 1 or 2, wherein the chondroitin sulfate-degrading enzyme is one or a plurality of enzymes selected from the group consisting of chondroitinase ABC, chondroitinase AC, and hyaluronidase.

4. The medicament according to Claim 1 or 2, wherein the chondroitin sulfate-degrading enzyme degrades chondroitin sulfate composed of a constituent disaccharide unit of chondroitin sulfate in which a hydroxyl group at 4-position of N-acetylgalactosamine is sulfated.

5. The medicament according to Claim 1 or 2, wherein the chondroitin sulfate-degrading enzyme is chondroitinase ABC or chondroitinase AC enzymes.

6. The medicament according to Claim 1 or 2, wherein the chondroitin sulfate-degrading enzyme is chondroitinase ABC.

7. The medicament according to any one of Claims 1 to 6, wherein the neural stem cell(s) and neural progenitor cell(s) are derived from a brain.

8. The medicament according to any one of Claims 1 to 6, wherein the neural stem cell(s) is a cultured neural stem cell(s) and neural progenitor cell(s) is a cultured neural progenitor cell(s).

9. The medicament according to any one of Claims 1 to 8, wherein the medicament is applied to a brain in the course of development to an adult brain.

10. The medicament according to any one of Claims 1 to 8, wherein the medicament is applied to a brain of a neonate or a newborn.

11. The medicament according to any one of Claims 1 to 10, which is an agent for ameliorating neonatal hypoxic-ischemic encephalopathy.

12. The medicament according to any one of Claims 1 to 10, which is an agent for reducing a cerebral infarction area.

13. The medicament according to any one of Claims 1 to 10, which is an agent for protecting a brain tissue.

14. An auxiliary agent for ameliorating brain damage induced by oxygen deficiency, which comprises a chondroitin sulfate-degrading enzyme as an active ingredient.

15. Use of a chondroitin sulfate-degrading enzyme and at least one of a neural stem cell(s) and a neural progenitor cell(s) in manufacturing the medicament according to any one of Claims 1 to 13.

16. Use of a chondroitin sulfate-degrading enzyme in manufacturing the auxiliary agent for ameliorating brain damage according to Claim 14.

17. A kit for ameliorating brain damage induced by oxygen deficiency, which comprises a chondroitin sulfate-degrading enzyme and at least one of a neural stem cell(s) and a neural progenitor cell(s).

18. A method of treating brain damage induced by oxygen deficiency, which comprises applying a chondroitin sulfate-degrading enzyme and at least one of a neural stem cell(s) and a neural progenitor cell(s) to a brain damage area.
